(19) Europäisches Patentamt
European Patent Office
Office européen des brevets

(11) EP 3 278 790 B1

(12) **EUROPEAN PATENT SPECIFICATION**

(45) Date of publication and mention of the grant of the patent:
**24.03.2021 Bulletin 2021/12**

(21) Application number: **16773421.9**

(22) Date of filing: **29.03.2016**

(51) Int Cl.:
***A61K 8/42*** *(2006.01)* ***A61K 8/34*** *(2006.01)*
***A61K 8/30*** *(2006.01)* ***A61K 31/166*** *(2006.01)*
***A61Q 19/08*** *(2006.01)* ***A23L 2/52*** *(2006.01)*
***A23L 33/15*** *(2016.01)* ***A23L 33/16*** *(2016.01)*
***A23L 33/155*** *(2016.01)* ***A23L 33/00*** *(2016.01)*

(86) International application number:
**PCT/KR2016/003219**

(87) International publication number:
**WO 2016/159640 (06.10.2016 Gazette 2016/40)**

(54) **ANTI-OXIDATIVE OR ANTI-AGING COMPOSITION CONTAINING 5-ADAMANTAN-1-YL-N-(2,4-DIHYDROXYBENZYL)-2,4-DIMETHOXYBENZAMIDE**

ANTIOXIDATIONS- ODER ANTI-AGING-MITTEL MIT 5-ADAMANTAN-1-YL-N-(2,4-DIHYDROXYBENZYL)-2,4-DIMETHOXYBENZAMID

COMPOSITION ANTI-OXYDANTE OU ANTI-VIEILLISSEMENT CONTENANT DU 5-ADAMANTAN-1-YL-N-(2,4-DIHYDROXYBENZYL)-2,4-DIMÉTHOXYBENZAMIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2015 KR 20150045397**

(43) Date of publication of application:
**07.02.2018 Bulletin 2018/06**

(73) Proprietor: **Amorepacific Corporation**
**Seoul 140-777 (KR)**

(72) Inventors:
- **JOO, Yung Hyup**
  **Yongin-si**
  **Gyeonggi-do 17074 (KR)**
- **BAEK, Heung Soo**
  **Yongin-si**
  **Gyeonggi-do 17074 (KR)**
- **HWANG, Jeong Ah**
  **Yongin-si**
  **Gyeonggi-do 17074 (KR)**
- **LEE, Hae Kwang**
  **Yongin-si**
  **Gyeonggi-do 17074 (KR)**
- **KIM, Yongjin**
  **Yongin-si**
  **Gyeonggi-do 17074 (KR)**
- **SHIN, Hong-Ju**
  **Yongin-si**
  **Gyeonggi-do 17074 (KR)**
- **LEE, Jon Hwan**
  **Yongin-si**
  **Gyeonggi-do 17074 (KR)**

(74) Representative: **Agasse, Stéphane et al**
**Cabinet GERMAIN & MAUREAU**
**B.P. 6153**
**69466 Lyon Cedex 06 (FR)**

(56) References cited:
**KR-A- 20130 015 954** **US-A1- 2014 234 241**

- **VAN THAI HA ET AL: "NF-kB/ AP-1-Targeted Inhibition of Macrophage-Mediated Inflammatory Responses by Depigmenting Compound AP 736 Derived from Natural 1,3-Diphenylpropane Skeleton", MEDIATORS OF INFLAMMATION., vol. 2014, 1 January 2014 (2014-01-01), pages 1-11, XP055331203, GB ISSN: 0962-9351, DOI: 10.1155/2014/354843**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- CHANG SEOK LEE ET AL: "A novel adamantyl benzylbenzamide derivative, AP736, suppresses melanogenesis through the inhibition of cAMP-PKA-CREB-activated microphthalmia-associated transcription factor and tyrosinase expression", EXPERIMENTAL DERMATOLOGY, vol. 22, no. 11, 29 November 2013 (2013-11-29), pages 762-764, XP055331206, COPENHAGEN; DK ISSN: 0906-6705, DOI: 10.1111/exd.12248
- C LEE ET AL: "769 AP736, a newly developed synthetic compound exerting whitening and anti-aging dual effect on skin cells", 76TH ANNUAL MEETING OF THE SOCIETY-FOR-INVESTIGATIVE-DERMATOLOGY (SID); PORTLAND, OR, USA, vol. 137, 1 May 2017 (2017-05-01), page S132, XP055506633, DOI: https://doi.org/10.1016/j.jid.2017.02.794
- HONG, Y. D. ET AL.: '3D-QSAR Study of Adamantyl N-benzylbenzamides as Melanogenesis Inhibitors' BIOORGANIC & MEDICINAL CHEMISTRY LETTERS vol. 24, no. 2, 2014, pages 667 - 673, XP055167578
- HA, V. T. ET AL.: 'NF-kB/ AP-1-Targeted Inhibition of Macrophage-Mediated Inflammatory Responses by Depigmenting Compound AP 736 Derived from Natural 1,3-Diphenylpropane Skeleton' MEDIATORS OF INFLAMMATION vol. 2014, 19 October 2014, pages 1 - 11, XP055331203
- BAEK, H. S. ET AL.: 'Adamantyl N-benzylbenz Amide: New Series of Depigmentation Agents with Tyrosinase Inhibitory Activity' BIOORGANIC & MEDICINAL CHEMISTRY LETTERS vol. 22, no. 5, 2012, pages 2110 - 2113, XP028459466
- LEE, C. S. ET AL.: 'A Novel Adamantyl Benzylbenzamide Derivative, AP 736, suppresses Melanogenesis through the Inhibition of cAMP-PKA-CREB-activated Microphthalmia -associated Transcription Factor and Tyrosinase Expression' EXPERIMENTAL DERMATOLOGY vol. 22, no. 11, 2013, pages 762 - 764, XP055331206

## Description

[Technical Field]

This specification relates to 5-adamantan-1-yl-N-(2,4-dihydroxybenzyl)-2,4-dimethoxybenzamide and a composition comprising the same for use of antioxidant and anti-aging.

[Background Art]

All organisms age while getting older, and the skin is the same. Efforts to delay such aging have been continued, and the questions on the nature and factor of aging have continuously arisen. Skin aging can be roughly classified into two types depending on the factors. Intrinsic aging, one of them, is that the structure and physiological function of the skin continuously deteriorate while aging, and extrinsic aging, the other, is caused by accumulated external stresses such as the rays of the sun. In particular, the rays of the sun are one of the well-known factors of aging, and the skin exposed to ultraviolet rays for a long time becomes thicker, collagen and elastin, the major constituents of the skin, are denatured, and the skin thus loses the elasticity. This aging of the skin is accompanied by various functional and structural changes. First of all, with regard to the structural changes of the skin due to aging, the thickness of the epidermis, dermis, and subcutaneous tissue constituting the skin become thinner. In addition, the extracellular matrix (ECM) component of the dermal tissue responsible for the elasticity and tensile of the skin is changed. ECM is composed of two major components. One of them is an elastic fiber which accounts for about 2 to 4% of the total ECM, and the other is collagen which accounts for about 70 to 80% of the total ECM. As aging progresses, the elasticity of the skin is greatly decreased by a decrease in the amount of collagen and elastin. These collagen and elastin are regulated by several factors, and collagen and elastin produced are decomposed by the expression of matrix metalloprotease such as collagenase and elastase, resulting in a decrease in the collagen content in the skin. When the amount of collagen and elastin decreases in the dermis, the epidermis of the skin becomes coarse and the elasticity of the skin decreases, which is the aging phenomenon. Various materials have been developed and used for the purpose of inhibiting a decrease in the amount of collagen and elastin. In particular, retinoids such as retinol and retinoic acid are known to improve wrinkles or elasticity (Dermatology therapy, 1998, 16, 357-364).

In addition, the relationship between aging and oxidation is well known by a number of studies, and particularly, wrinkle formation and localized blackening caused by skin aging are also known to be closely related to such oxidation phenomena. Even at the same age, some people look much younger than their age and some people look much older than their actual age. The causes of aging such as skin wrinkle formation and localized blackening can be classified into an intrinsic factor such as an increase in age, a genetic factor by the shapes of the skeleton and muscles, the sustained relaxation and contraction of the muscles, and an environmental factor by environmental pollutants, stress, and the like. By the skin aging phenomenon caused by various factors described above, the number of wrinkles due to decreased keratinocyte and fibroblast cleavage, decreased collagen synthesis, increased MMP production, the increased signal transmission of melanin production, and the like increases, the elasticity of the skin decreases, and the stain, freckles, and age spots increase. In particular, recent studies have shown that in the case of healthy skin, free radicals are removed by a biological antioxidant defense such as superoxide dismutase and catalase, but when this removal is incomplete, oxidation by active oxygen (a kind of free radical) occurs and the functions of skin cells and tissues deteriorate, resulting in accelerated skin wrinkle formation and localized blackening. The production of such active oxygen involved in strong biological oxidation is accelerated by external factors (pollution, stress) and internal factors (energy metabolism in vivo). Active oxygen bonds with lipid to form a harmful substance called peroxide lipid in some cases. The peroxide lipid acts on blood vessels and causes various diseases including arteriosclerosis and thrombosis. In addition, it has been reported that a decrease in the ability to produce collagen to be closely related to healthy skin and an increase in abnormal tangling phenomenon of these collagen fibers lead to a remarkable decrease in skin elasticity, and thus a remarkable decrease in the production amount of hyaluronic acid and glycosaminoglycan of a kind of glycoprotein, which are responsible for skin moisturization are caused.

The compound 5-adamantan-1-yl-N-(2,4-dihydroxybenzyl)-2,4-dimethoxybenzamide (also known as AP376), has been disclosed in the patent application US 2014/234241 A1, as well as in some publications (Lee et al (2013), Experimental Dermatology, 22(11), 762-764 ; Ha et al (2014), Mediators of Inflammation, 2014, 1-11). This compound has been shown to have anti-melanogenic and anti-inflammatory effects, but no effect on anti-aging had been described.

[Citation List]

[Patent Literature] Korean Patent Publication No. 10-2013-0015954

[Summary of Invention]

[Technical Problem]

In an aspect, an object of the present invention is to provide a composition exhibiting excellent any one or more activities of antioxidation and anti-aging without toxicity.

[Solution to Problem]

In an aspect, the present invention provides an antioxidant or anti-aging composition containing 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof as an active ingredient.

In an embodiment, the 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof can scavenge a free radical.

In another embodiment, the 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof can inhibit the expression of matrix metalloprotease-1 (MMP-1).

In an aspect, the composition of the present invention provides an antioxidant or anti-aging composition that is a cosmetic or food composition.

[Advantageous Effects of Invention]

In an aspect of the present invention, the composition containing 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof as an active ingredient can exhibit an excellent antioxidant or anti-aging effect without toxicity.

The antioxidant composition according to an aspect of the present invention has an effect of scavenging a free radical.

The anti-aging composition according to an aspect of the present invention has an effect of inhibiting the expression of matrix metalloprotease-1 (MMP-1).

[Brief Description of Drawings]

FIG. 1 is a graph illustrating the cell viability of 5-adamantan-1-yl-N-(2,4-dihydroxybenzyl)-2,4-dimethoxybenzamide (referred to as 'AP736' in FIG. 1) in Raw 264.7 cells.

FIG. 2 is a graph illustrating the antioxidant effect of 5-adamantan-1-yl-N-(2,4-dihydroxybenzyl)-2,4-dimethoxybenzamide (referred to as 'AP736' in FIG. 2) measured by a DPPH method.

FIG. 3 is a graph illustrating the percent MMP-1 expression measured in order to confirm the anti-aging effect of 5-adamantan-1-yl-N-(2,4-dihydroxybenzyl)-2,4-dimethoxybenzamide (referred to as 'AP736' in FIG. 3) (RA in FIG. 3 is retinoic acid).

[Description of Embodiments]

In an aspect of the present invention, an antioxidant composition containing 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof as an active ingredient is provided. Specifically, the antioxidation may be skin antioxidation.

In an aspect of the present invention, as a method for enhancing antioxidation of a subject, the method comprising a step of administering an effective dosage of 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof to a subject in need thereof is provided.

In an aspect of the present invention, 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof for enhancing antioxidation is provided.

Specifically, the antioxidation may be skin antioxidation.

In an embodiment, an antioxidant composition in which 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof scavenges free radicals is provided.

In another aspect of the present invention, an anti-aging composition containing 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof as an active ingredient is provided.

In another aspect of the present invention, as a method for enhancing anti-aging of a subject, the method comprising a step of administering an effective dosage of 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof to a subject in need thereof is provided.

In another aspect of the present invention, a non-therapeutic use of a compound 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof for enhancing anti-aging is provided.

In an embodiment, an anti-aging composition in which 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof inhibits the expression of matrix metalloprotease-1 (MMP-1) is provided.

Specifically, the anti-aging may be skin anti-aging.

In still another aspect of the present invention, a composition for enhancing skin elasticity or improving wrinkle comprising 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof as an active ingredient is provided.

In still another aspect of the present invention, as a method for enhancing skin elasticity or improving wrinkles of a subject, the method comprising a step of administering an effective dosage of 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof to a subject in need thereof is provided.

In still another aspect of the present invention, 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof for enhancing skin elasticity or improving wrinkles is provided.

In another aspect of the present invention, the antioxidant, anti-aging, or skin elasticity enhancing or wrinkle improving composition provides an antioxidant, anti-aging, or skin elasticity enhancing or wrinkle improving composition that is a cosmetic or food composition.

In an embodiment of the present invention, the present invention is a composition containing 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof as an active ingredient.

A method for preparing a compound represented by the following Chemical Formula 1 is as follows.

[Chemical Formula 1]

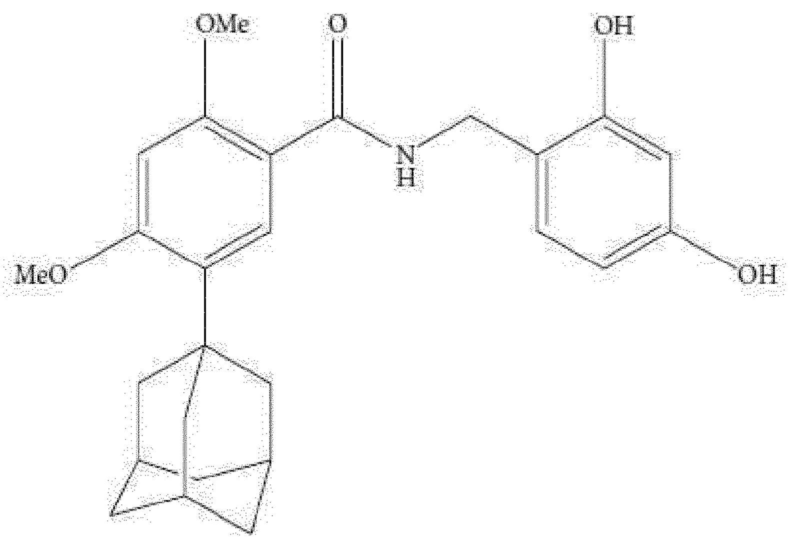

A method for preparing a benzamide compound substituted with an adamantane group comprises

i) a step of synthesizing adamantanyl-hydroxybenzoic acid by reacting hydroxybenzoic acid with an adamantane compound in the presence of an acid catalyst;
ii) a step of synthesizing an adamantanyl-alkoxybenzoic acid by reacting adamantanyl-hydroxybenzoic acid with an

alkylsulfate; and

iii) a step of synthesizing a benzamide compound substituted with an adamantane group by reacting the adamantanyl-alkoxybenzoic acid with an alkylphenylamine substituted with a hydroxy group.

The method for preparing a benzamide compound substituted with an adamantane group can be illustrated by the following Reaction Formula 1.

[Reaction Formula 1]

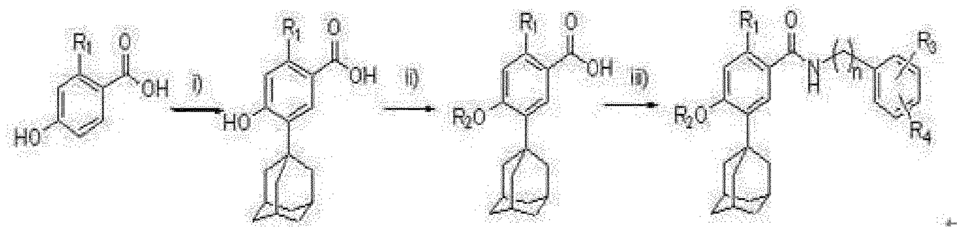


In Reaction Formula 1 above,
R1, R3, and R4 are each independently selected from the group consisting of hydrogen, hydroxy, C1 to C5 alkoxy, C3 to C6 cycloalkoxy, aryloxy, and C1 to C5 haloalkoxy,
R2 is selected from the group consisting of hydrogen, C1 to C5 alkyl, C3 to C6 cycloalkyl, aryl, and C1 to C5 haloalkyl, and n is an integer selected from 1 to 5.

The method for preparing a benzamide compound substituted with an adamantane group may comprise

i) a step of synthesizing adamantanyl-dihydroxybenzoic acid by reacting dihydroxybenzoic acid with an adamantane compound in the presence of an acid catalyst;

ii) a step of synthesizing adamantanyl-hydroxy-alkoxybenzoic acid or adamantanyl-dialkoxybenzoic acid by reacting adamantanyl-dihydroxybenzoic acid with a dialkyl sulfate in the presence of a hydroxide; and

iii) a step of synthesizing a benzamide compound substituted with an adamantane group by reacting adamantanyl-hydroxy-alkoxybenzoic acid or adamantanyl-dialkoxybenzoic acid with benzylamine or phenethylamine which is substituted with a hydroxy group.

The method for preparing a benzamide compound substituted with an adamantane group may comprise

i) a step of synthesizing 5-adamantanyl-2,4-dihydroxybenzoic acid by reacting 2,4-dihydroxybenzoic acid with 1-adamantanol in a dichloromethane solvent in the presence of acetic acid and sulfuric acid catalysts at room temperature;

ii) a step of synthesizing 5-adamantanyl-2-hydroxy-4-methoxybenzoic acid or 5-adamantanyl-2,4-dimethoxybenzoic acid by reacting 5-adamantanyl-2,4-dihydroxybenzoic acid with dimethyl sulfate in the presence of sodium hydroxide or potassium hydroxide; and

iii) a step of synthesizing a benzamide compound substituted with an adamantane group by reacting 5-adamantanyl-2-hydroxy-4-methoxybenzoic acid or 5-adamantanyl-2,4-dimethoxybenzoic acid with benzylamine or phenethylamine which is substituted with a hydroxy group in the presence of N-hydroxysuccinimide (HOSu) and N,N'-dicyclohexylcarbodiimide (DCC).

5-Adamantan-1-yl-N-(2,4-dihydroxybenzyl)-2,4-dimethoxybenzamide prepared through the above process, an isomer thereof, a pharmaceutically acceptable salt thereof, a hydrate thereof, or a solvate thereof has an antioxidant and anti-aging effects.

In an aspect of the present invention, a composition containing 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof having antioxidant and anti-aging effects as an active ingredient is provided.

The composition may contain from 0.01wt% to 20wt% of the compound, an isomer thereof, a pharmaceutically acceptable salt thereof, a hydrate thereof, or a solvate thereof, based on the total weight of the composition.

From a perspective, the 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof may be contained at 0.01wt% or more, 0.02wt% or more, 0.03wt% or more, 0.04wt% or more, 0.05wt% or more, 0.1wt% or more, 0.2wt% or more, 0.3wt%

or more, 0.4wt% or more, 0.5wt% or more, 0.6wt% or more, 0.7wt% or more, 0.8wt% or more, 0.9wt% or more, 1.0wt% or more, 2.0wt% or more, 3.0wt% or more, 4.0wt% or more, 4.1wt% or more, 4.2wt% or more, 4.3wt% or more, 4.4wt% or more, 4.5wt% or more, 4.6wt% or more, 4.7wt% or more, 4.8wt% or more, 4.9wt% or more, or 5.0wt% or more based on the total weight of the composition.

From a perspective, the 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof may be contained at 20wt% or less, 19.5wt% or less, 19wt% or less, 18wt% or less, 17wt% or less, 16wt% or less, 15wt% or less, 14wt% or less, 13wt% or less, 12wt% or less, 11wt% or less, 10wt% or less, 9wt% or less, 8wt% or less, 7wt% or less, 6wt% or less, 5.9wt% or less, 5.8wt% or less, 5.7wt% or less, 5.6wt% or less, 5.5wt% or less, 5.4wt% or less, 5.3wt% or less, 5.2wt% or less, or 5.1wt% or less based on the total weight of the composition.

When the 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof is contained within the above range, the composition is not only suitable for exhibiting the intended effect of the present invention but also both the stability and safety of the composition can be satisfied. It may be appropriate to contain 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof within the above range from the viewpoint of cost-effectiveness. Specifically, when 5-adamantan-1-yl-N-(2,4-dihydroxybenzyl)-2,4-dimethoxybenzamide, an isomer thereof, a pharmaceutically acceptable salt thereof, a hydrate thereof, or a solvate thereof is contained within the above range, the antioxidant and anti-aging effects can be effectively obtained without toxicity.

In an embodiment, the composition may be a cosmetic or food composition.

The composition may also be used in the form of a pharmaceutically acceptable salt thereof. The salt is not particularly limited as long as it is pharmaceutically acceptable, for example, hydrochloric acid, sulfuric acid, nitric acid, phosphoric acid, hydrofluoric acid, hydrobromic acid, formic acid acetic acid, tartaric acid, lactic acid, citric acid, fumaric acid, maleic acid, succinic acid, methanesulfonic acid, benzenesulfonic acid, toluenesulfonic acid, naphthalenesulfonic acid, and the like may be used.

The composition according to the present invention may be administered to mammals such as rats, mice, livestock, and humans.

In an aspect of the present invention, the food composition may be a health functional food composition.

The formulation of the food composition according to this specification is not particularly limited, but the food composition may be formulated into, for example, tablets, granules, powders, liquid preparations such as drinks, caramels, gels, bars, and the like. Components commonly used in the field other than the active ingredient may be appropriately selected and blended with the food composition of each formulation by those skilled in the art depending on the purpose of formulation or use without difficulty. A synergistic effect can be obtained when the food composition is simultaneously applied with other raw materials.

In the food composition according to this specification, the dosage determination of the active ingredient is determined by those skilled in the art. The daily dosage thereof may be, for example, from 0.1 mg/kg/day to 5000 mg/kg/day and more specifically from 1 mg/kg/day to 500 mg/kg/day, but it is not limited thereto and may vary depending on various factors such as the age, health condition, complications, and the like of the subject to be administered.

The food composition according to this specification may be, for example, various foodstuffs such as chewing gum, caramel product, candy, ice cream, confectionery, and the like, beverages such as soft drinks, mineral water, alcoholic beverages, and the like, and health functional foods including vitamins, minerals, and the like.

In addition to the above, the food composition of an aspect of the present invention may contain various nutrients, vitamins, minerals (electrolytes), flavors such as synthetic flavorings and natural flavorings, colorants and enhancers (cheese, chocolate, and the like), pectic acid and salts thereof, alginic acid and salts thereof, organic acids, protective colloid thickeners, pH adjusting agents, stabilizers, preservatives, glycerin, alcohols, and carbonating agents used in carbonated beverages. In addition, the functional food compositions of the present invention may contain natural fruit juice and flesh for the production of fruit juice drinks and vegetable drinks. These components may be used independently or in combination. The proportion of such additives is not so critical, but the additives are generally contained in the range of from 0 to about 20 parts by weight per 100 parts by weight of the composition of the present invention.

The formulation of the cosmetic composition is not particularly limited and may be appropriately selected depending on the purpose. For example, the cosmetic composition may be formulated into one or more selected from the group consisting of a skin lotion, a skin softener, a skin toner, an astringent, a lotion, a milk lotion, a moisturizing lotion, a nourishing lotion, a massage cream, a nourishing cream, a moisturizing cream, a hand cream, a foundation, an essence, a nourishing essence, a pack, a soap, a cleansing foam, a cleansing lotion, a cleansing cream, a body lotion, and a body cleanser, but the formulation is not limited thereto.

In a case in which the formulation of the present invention is a paste, a cream, or a gel, animal fibers, plant fibers, wax, paraffin, starch, tracant, cellulose derivatives, polyethylene glycol, silicone, bentonite, silica, talc, zinc oxide, or the like may be used as a carrier component.

In a case in which the formulation of the present invention is a powder or a spray, lactose, talc, silica, aluminum hydroxide, calcium silicate, or polyamide powder may be used as a carrier component, and particularly in the case of a spray, propellants such as chlorofluorohydrocarbons, propane/butane, or dimethyl ether may be further contained.

In a case in which the formulation of the present invention is a solution or an emulsion, solvents, dissolvents, or emulsifiers are used as a carrier component, and examples thereof may include water, ethanol, isopropanol, ethyl carbonate, ethyl acetate, benzyl alcohol, benzyl benzoate, propylene glycol, 1,3-butyl glycol oil, glycerol aliphatic esters, polyethylene glycol, or sorbitan fatty acid esters.

In a case in which the formulation of the present invention is a suspension, liquid diluents such as water, ethanol, or propylene glycol, suspending agents such as ethoxylated isostearyl alcohol, polyoxyethylene sorbitol ester, and polyoxyethylene sorbitan ester, microcrystalline cellulose, aluminum metahydroxide, bentonite, agar, tracant, or the like may be used as a carrier component.

In a case in which the formulation of the present invention is a surfactant-containing cleansing, aliphatic alcohol sulfate, aliphatic alcohol ether sulfate, sulfosuccinic acid monoester, isethionate, imidazolinium derivatives, methyltaurate, sarcosinate, fatty acid amide ether sulfate, alkylamidobetaines, aliphatic alcohols, fatty acid glycerides, fatty acid diethanolamides, vegetable oils, linolenic derivatives, ethoxylated glycerol fatty acid esters, or the like may be used as a carrier component.

The content of the active ingredient is not particularly limited, but it may be from 0.01 to 20wt% based on the total weight of the composition. Excellent effects can be exhibited without side effects in a case in which the active ingredient satisfies the above-mentioned content range.

The cosmetic composition may further contain functional additives and components to be contained in a general cosmetic composition. As the above-mentioned functional additives, components selected from the group consisting of water-soluble vitamins, oil-soluble vitamins, polymeric peptides, polymeric polysaccharides, sphingolipids, and seaweed extracts may be contained.

The cosmetic composition of the present invention may further contain components to be contained in a general cosmetic composition together with the above-described functional additives. Examples of the components to be additionally blended may include fats and oils, moisturizers, emollients, surfactants, organic and inorganic pigments, organic powders, ultraviolet absorbers, preservatives, antiseptics, antioxidants, plant extracts, pH, alcohols, colorants, fragrances, blood circulation accelerators, coolants, antiperspirants, purified water, and the like.

[Embodiments]

Hereinafter, the configuration and effects of the present invention will be described in more detail with reference to Examples and Experimental Examples. These Examples and Experimental Examples are provided for illustrative purposes only in order to facilitate understanding of the present invention.

[Example] Preparation of 5-adamantan-1-yl-N-(2,4-dihydroxybenzyl)-2,4-dimethoxybenzamide

5-Adamantan-1-yl-2,4-dimethoxybenzoic acid (0.634 g), N-hydroxysuccinimide (0.24 g), and N,N'-dicyclohexylcarbodiimide (0.43 g) are dissolved in dioxane (10 mL) and stirred for 12 hours. The resulting solid was filtered and the filtrate was added dropwise to a mixed solution of 2,4-dihydroxybenzylamine bromate (0.54 g), sodium bicarbonate (0.18 g), and water (2 mL) and stirred at 50°C for 2 hours. After the reaction is completed, the solution is cooled to room temperature, neutralized with a 10% HCl solution, and washed with ethyl acetate (50 mL). The organic solution layer is dried over anhydrous magnesium sulfate, filtered, concentrated under reduced pressure, separated by column chromatography to obtain 0.14 g of the title compound as a white solid.

As a result of NMR analysis of the above white solid, it was confirmed that the white solid was 5-adamantan-1-yl-N-(2,4-dihydroxybenzyl)-2,4-dimethoxybenzamide through the following signals.

$^1$H NMR(300MHz, DMSO-d6) $\delta$ 9.67 (s, 1H), 9.13 (s, 1H), 8.51 (m, 1H), 7.78 (m,1H), 6.92 (d, 1H, J = 8.1 Hz), 6.66 (s, 1H), 6.27 (s, 1H), 6.16 (d, 1H, J = 8.1 Hz), 4.30 (d, 2H, J = 5.4 Hz), 3.93 (s, 3H), 3.88 (s, 3H), 1.98 (s, 9H), 1.71 (s, 6H)

Hereinafter, the effects of the present invention will be described with reference to various Experimental Examples, but these Experimental Examples are illustrative only and do not limit the scope of the invention.

Cell incubation

RAW264.7 cells (obtained from ATCC (Rockville, MD, USA) as a murine macrophage cell line) were incubated in RPMI 1640 medium supplemented with FBS (10% heat-inactivated fetal bovine serum; Gibco, Grand Island, NY, USA), glutamine, antibiotics (penicillin and streptomycin) at 37°C and 5% $CO_2$.

In all experiments, cells were detached with a cell scraper. When cells were incubated at a concentration of 2 $\times 10^6$ cells/mL in the experiment, the percentage of dead cells was 1% or less (confirmed by a trypan blue dye exclusion

method).

[Experimental Example 1] Cytotoxicity test of 5-adamantan-1-yl-N-(2,4-dihydroxybenzyl)-2,4-dimethoxybenzamide

In order to determine the appropriate concentration of extract treatment for anti-inflammatory evaluation of 5-adamantan-1-yl-N-(2,4-dihydroxybenzyl)-2,4-dimethoxybenzamide, the cytotoxicity of 5-adamantan-1-yl-N-(2,4-dihydroxybenzyl)-2,4-dimethoxybenzamide at each concentration was evaluated in the cells used in the experiments.

Raw 264.7 cells in the subculture were plated on a well plate at a density of $1 \times 10^6$ cells/well. After 18 hours of cell incubation, the cells were subjected to a sample treatment so that the concentration of 5-adamantan-1-yl-N-(2,4-dihydroxybenzyl)-2,4-dimethoxybenzamide (referred to as 'AP736' in FIG. 1) was from 0 to 30 μM. The cells were incubated for 8 hours or 24 hours in an incubator. The cytotoxic effect was evaluated by the conventional MTT method. Before the last 3 hours of incubation, 10 μL of MTT {3-[4,5-dimethylthiazol-2-yl]-2,5-diphenyltetrazolium bromide} solution (10 mg/mL in phosphate-buffered saline, pH 7.4) was added to each well. To each well, 15% SDS (sodium dodecyl sulfate) was added to terminate the reaction, and the formazan crystal was then dissolved therein. Thereafter, the absorbance at 570 nm was measured on a microplate reader.

The results are illustrated in FIG. 1. 5-Adamantan-1-yl-N-(2,4-dihydroxybenzyl)-2,4-dimethoxybenzamide did not exhibit toxicity to Raw 264.7 cells for 8 hours and 24 hours at concentrations of 10 and 20 μM, and a cell viability of about 100% was thus confirmed. A cell viability of about 80% was confirmed when the cells were incubated for 24 hours at a concentration of 30 μM.

[Experimental Example 2] Evaluation on antioxidant effect of 5-adamantan-1-yl-N-(2,4-dihydroxybenzyl)-2,4-dimethoxybenzamide (DPPH evaluation)

The DPPH free radical scavenging evaluation method was performed as follows.

In 5-adamantan-1-yl-N-(2,4-dihydroxybenzyl)-2,4-dimethoxybenzamide (referred to as 'AP736' in FIG. 2) prepared in Example, a reaction product adjusted to a concentration of from 0 to 200 μg/ml and a methanol solution of DPPH (0.2 mM) were incubated for 30 minutes. The absorbance of the solution was measured at 517 nm. The scavenging ability was expressed by the value obtained by comparing the absorbance of the solution to that (100%) of the control DPPH solution in % as presented in the following Mathematical Formula 1.

[Mathematical Formula 1]

$$Inhibition\,(\%) = \left\{1 - \left[\frac{(A_{Experiment} - A_{Blank})}{A_{Control}}\right]\right\} \times 100$$

The measurement results are illustrated in FIG. 2. The y-axis represents the percentage of DPPH radical scavenging activity and the x-axis represents the concentration of the sample. The percentage of radical scavenging activity increases as the concentration of 5-adamantan-1-yl-N-(2,4-dihydroxybenzyl)-2,4-dimethoxybenzamide (referred to as 'AP736' in FIG. 2) increases. As a reference material, vitamin C of a powerful antioxidant was used. As a result of the experiment, it was determined that 5-adamantan-1-yl-N-(2,4-dihydroxybenzyl)-2,4-dimethoxybenzamide (referred to as 'AP736' in FIG. 2) was able to act as an antioxidant. In particular, 5-adamantan-1-yl-N-(2,4-dihydroxybenzyl)-2,4-dimethoxybenzamide is expected to have an advantage over vitamin C in terms of stability.

[Experimental Example 3] Evaluation on anti-aging effect of 5-adamantan-1-yl-N-(2,4-dihydroxybenzyl)-2,4-dimethoxybenzamide (MMP-1 assay)

In order to confirm the anti-aging effect of the sample, an experiment to see how much 5-adamantan-1-yl-N-(2,4-dihydroxybenzyl)-2,4-dimethoxybenzamide can inhibit the expression of matrix metalloprotease-1 (MMP-1) to be induced by ultraviolet (UV) rays was performed.

Human fibroblasts (HS68 cells, purchased from ATCC (Rockville, MD, USA)) were plated on a well plate at a density of $7.5 \times 10^4$ cells/well, then incubated in DMEM (Dulbecco's Modified Eagle's Medium) containing 10% FBS for 24 hours, then washed with PBS (phosphate-buffered saline), then 0.5 ml of PBS was added thereto, and the expression of MMP-1 was induced by irradiating the resultant incubated cells with ultraviolet rays. After changing to Serum-Free DMEM, the resultant was treated with 5-adamantan-1-yl-N-(2,4-dihydroxybenzyl)-2,4-dimethoxybenzamide (referred to

as 'AP736' in FIG. 3) prepared in Example. The amount of the supernatant obtained after incubation for a certain period of time (48 hours) was measured by using an MMP-1 ELISA kit.

In other words, HS68 cells of human dermal fibroblasts were treated with ultraviolet rays to increase the expression of MMP-1, and the resulting HS68 cells were then treated with each sample to see whether the expression of MMP-1 decreased.

The experimental results are illustrated in FIG. 3. The x-axis represents an increase in expression of MMP-1 in the control group (reference, 100% on the y-axis) and HS68 cells (200%) treated with ultraviolet rays (NT). The expression of MMP-1 decreased in HS68 cells treated with retinoic acid (RA) which was known to inhibit the expression of MMP-1, and similarly, the expression of MMP-1 tended to decrease when the concentration of 5-adamantan-1-yl-N-(2,4-dihydroxybenzyl)-2,4-dimethoxybenzamide (referred to as 'AP736' in FIG. 3) was increased to 0.1, 0.5, and 1 μM. In other words, a result was obtained that 5-adamantan-1-yl-N-(2,4-dihydroxybenzyl)-2,4-dimethoxybenzamide (referred to as 'AP736' in FIG. 3) inhibited the expression of MMP-1 in the range in which cytotoxicity was not exhibited as confirmed in Experimental Example 1.

Hereinafter, as described above, Formulation Examples of a composition having antioxidant or anti-aging effect according to an aspect of the present invention will be described. However, the present invention can be applied to various other formulations, and Formulation Examples are not intended to limit the present invention but only to illustrate the present invention.

[Formulation Example 1] Health food

5-Adamantan-1-yl-N-(2,4-dihydroxybenzyl)-2,4-dimethoxybenzamide ·· · 10 mg
Vitamin mixture
Vitamin A acetate ··· 70 μg
Vitamin E ··· 1.0 mg
Vitamin B1 ··· 0.13 mg
Vitamin B2 ··· 0.15 mg
Vitamin B6 ··· 0.5 mg
Vitamin B12 ··· 0.2 μg
Vitamin C ··· 10 mg
Biotin ··· 10 μg
Nicotinic amide ··· 1.7 mg
Folic acid ··· 50 μg
Calcium pantothenate ··· 0.5 mg
Mineral mixture
Ferrous sulfate ··· 1.75 mg
Zinc oxide ··· 0.82 mg
Magnesium carbonate ··· 25.3 mg
Potassium dihydrogen phosphate ··· 15 mg
Calcium hydrogen phosphate ··· 55 mg
Potassium citrate ··· 90 mg
Calcium carbonate ... 100 mg
Magnesium chloride ··· 24.8 mg

As the composition ratio of the above-mentioned vitamin and mineral mixtures, components suitable for health food are mixed in a preferred embodiment, but the blending ratio thereof may be arbitrarily modified.

[Formulation Example 2] Health drinks

5-Adamantan-1-yl-N-(2,4-dihydroxybenzyl)-2,4-dimethoxybenzamide ·· · 10 mg
Citric acid ... 1000 mg
Oligosaccharide ··· 100 g
Taurine ··· 1 g
Purified water ··· balance

The above components are mixed according to a conventional health drink manufacturing method, and the mixture is stirred and heated at 85°C for about 1 hour, and then the solution thus prepared is filtered and sterilized.

[Formulation Example 3] Tablets

After 1 mg of 5-adamantan-1-yl-N-(2,4-dihydroxybenzyl)-2,4-dimethoxybenzamide, 50 mg of soybean extract,

100 mg of glucose, 50 mg of red ginseng extract, 96 mg of starch, and 4 mg of magnesium stearate were mixed together, 40 mg of 30% ethanol was added to the mixture to form granules, followed by drying at 60°C and tableting using a tablet machine.

[Formulation Example 4] Granules

After 1 mg of 5-adamantan-1-yl-N-(2,4-dihydroxybenzyl)-2,4-dimethoxybenzamide, 50 mg of soybean extract, 100 mg of glucose, and 600 mg of starch were mixed together, 100 mg of 30% ethanol is added to the mixture to form granules, followed by drying at 60°C to form granules, and the granules are then filled in a capsule.

[Formulation Example 5] Cosmetic water

A cosmetic water is prepared according to a conventional method to have the composition presented in the following Table 1.

[Table 1]

| Components | Content (wt%) |
|---|---|
| 5-Adamantan-1-yl-N-(2,4-dihydroxybenzyl)-2,4-dimethoxybenzamide | 0.1 |
| Glycerin | 3.0 |
| Butylene glycol | 2.0 |
| Propylene glycol | 2.0 |
| Carboxyvinyl polymer | 0.1 |
| PEG 12 nonyl phenyl ether | 0.2 |
| Polysorbate 80 | 0.4 |
| Ethanol | 10.0 |
| Triethanolamine | 0.1 |
| Preservative, colorant, and fragrance | Proper amounts |
| Purified water | Balance |

[Formulation Example 6] Nourishing cream

A nourishing cream is prepared according to a conventional method to have the composition presented in the following Table 2.

[Table 2]

| Components | Content (wt%) |
|---|---|
| 5-Adamantan-1-yl-N-(2,4-dihydroxybenzyl)-2,4-dimethoxybenzamide | 2.0 |
| Polysorbate 60 | 1.5 |
| Sorbitan sesquioleate | 0.5 |
| PEG 60 hardened castor oil | 2.0 |
| Liquid paraffin | 10.0 |
| Squalane | 5.0 |
| Caprylic/capric triglyceride | 5.0 |
| Glycerin | 5.0 |
| Butylene glycol | 3.0 |
| Propylene glycol | 3.0 |

(continued)

| Components | Content (wt%) |
|---|---|
| Triethanolamine | 0.2 |
| Preservative, colorant, and fragrance | Proper amounts |
| Purified water | Balance |

[Formulation Example 7] Massage cream

A massage cream is prepared according to a conventional method to have the composition presented in the following Table 3.

[Table 3]

| Components | Content (wt%) |
|---|---|
| 5-Adamantan-1-yl-N-(2,4-dihydroxybenzyl)-2,4-dimethoxybenzamide | 1.0 |
| Beeswax | 10.0 |
| Polysorbate 60 | 1.5 |
| PEG 60 hardened castor oil | 2.0 |
| Sorbitan sesquioleate | 0.8 |
| Liquid paraffin | 40.0 |
| Squalane | 5.0 |
| Caprylic/capric triglyceride | 4.0 |
| Glycerin | 5.0 |
| Butylene glycol | 3.0 |
| Propylene glycol | 3.0 |
| Triethanolamine | 0.2 |
| Preservative, colorant, and fragrance | Proper amounts |
| Purified water | Balance |

[Formulation Example 8] Pack

A pack is prepared according to a conventional method to have the composition presented in the following Table 4.

[Table 4]

| Components | Content (wt%) |
|---|---|
| 5-Adamantan-1-yl-N-(2,4-dihydroxybenzyl)-2,4-dimethoxybenzamide | 0.2 |
| Polyvinyl alcohol | 13.0 |
| Sodium carboxymethylcellulose | 0.2 |
| Glycerin | 5.0 |
| Allantoin | 0.1 |
| Ethanol | 6.0 |
| PEG 12 nonyl phenyl ether | 0.3 |
| Polysorbate 60 | 0.3 |

(continued)

| Components | Content (wt%) |
|---|---|
| Preservative, colorant, and fragrance | Proper amounts |
| Purified water | Balance |

[Formulation Example 9] Gel

A gel is prepared according to a conventional method to have the composition presented in the following Table 5.

[Table 5]

| Components | Content (wt%) |
|---|---|
| 5-Adamantan-1-yl-N-(2,4-dihydroxybenzyl)-2,4-dimethoxybenzamide | 0.5 |
| Ethylenediamine sodium acetate | 0.05 |
| Glycerin | 5.0 |
| Carboxyvinyl polymer | 0.3 |
| Ethanol | 5.0 |
| PEG 60 hardened castor oil | 0.5 |
| Triethanolamine | 0.3 |
| Preservative, colorant, and fragrance | Proper amounts |
| Purified water | Balance |

[Formulation Example 10] Ointment

An ointment was prepared according to a conventional method to have the composition presented in the following Table 6.

[Table 6]

| Components | Content (wt%) |
|---|---|
| 5-Adamantan-1-yl-N-(2,4-dihydroxybenzyl)-2,4-dimethoxybenzamide | 1.5 |
| Glycerin | 8.0 |
| Butylene glycol | 4.0 |
| Liquid paraffin | 15.0 |
| β-glucan | 7.0 |
| Carbomer | 0.1 |
| Caprylic/capric triglyceride | 3.0 |
| Squalane | 1.0 |
| Cetearyl glucoside | 1.5 |
| Sorbitan stearate | 0.4 |
| Cetearyl alcohol | 1.0 |
| Beeswax | 4.0 |
| Preservative, colorant, and fragrance | Proper amounts |
| Purified water | Balance |

## Claims

1. A non-therapeutic use of a compound 5-adamantan-1-yl-N-(2,4-dihydroxybenzyl)-2,4-dimethoxybenzamide represented by the following Chemical Formula 1, an isomer thereof, a pharmaceutically acceptable salt thereof, a hydrate thereof, or a solvate thereof for enhancing anti-aging,
wherein the compound, an isomer thereof, a pharmaceutically acceptable salt thereof, a hydrate thereof, or a solvate thereof inhibits expression of matrix metalloprotease-1 (MMP-1), and is contained in a cosmetic or food composition.

[Chemical Formula 1]

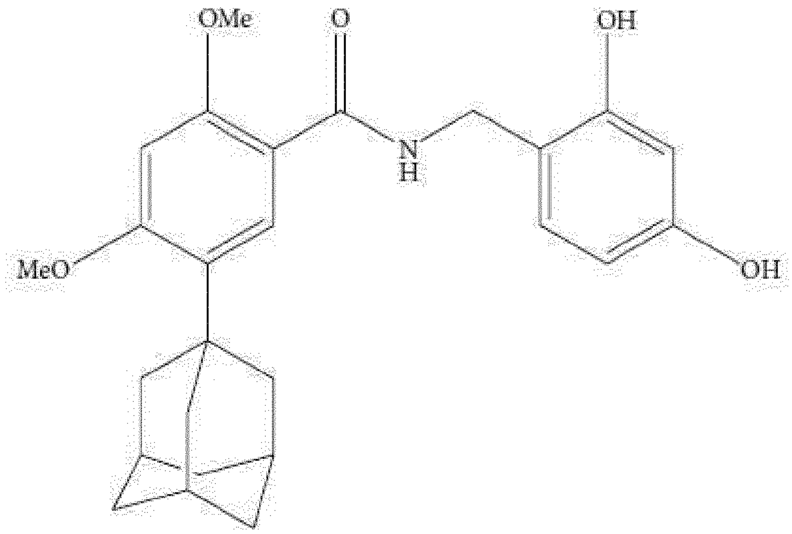

2. A non-therapeutic use of a compound 5-adamantan-1-yl-N-(2,4-dihydroxybenzyl)-2,4-dimethoxybenzamide represented by the following Chemical Formula 1, an isomer thereof, a pharmaceutically acceptable salt thereof, a hydrate thereof, or a solvate thereof for enhancing skin elasticity or improving wrinkle,
wherein the compound, an isomer thereof, a pharmaceutically acceptable salt thereof, a hydrate thereof, or a solvate thereof inhibits expression of matrix metalloprotease-1 (MMP-1), and is contained in a cosmetic or food composition.

[Chemical Formula 1]

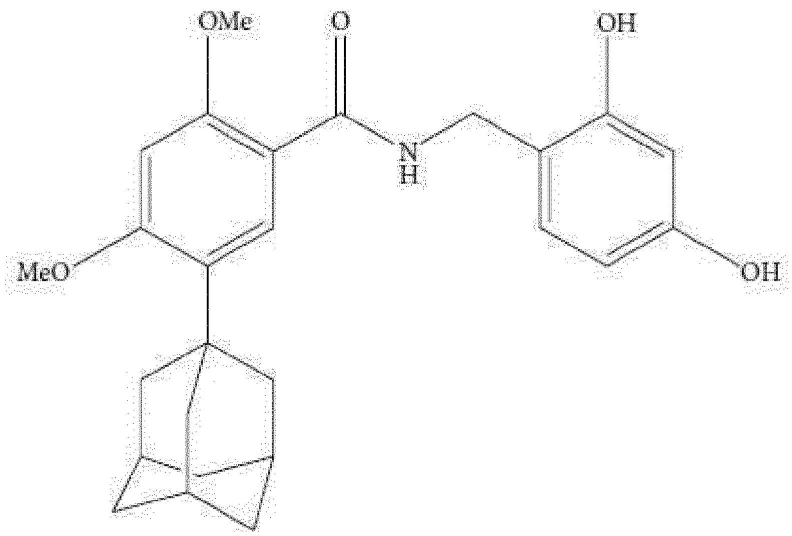

3. The non-therapeutic use according to claim 2, wherein the compound, an isomer thereof, a pharmaceutically acceptable salt thereof, a hydrate thereof, or a solvate thereof enhances collagen or elastin.

4. The non-therapeutic use according to claim 2 or claim 3, wherein the compound, an isomer thereof, a pharmaceutically acceptable salt thereof, a hydrate thereof, or a solvate thereof is contained in a concentration of 0.01wt% to 20wt% based on a total weight of the composition.

**Patentansprüche**

1. Nichttherapeutische Verwendung einer Verbindung 5-Adamantan-1-yl-N-(2,4-dihydroxybenzyl)-2,4-dimethoxybenzamid, durch die folgende chemische Formel 1 dargestellt, eines Isomers davon, eines pharmazeutisch annehmbaren Salzes davon, eines Hydrats davon oder eines Solvats davon zur Förderung von Alterungsschutz, wobei die Verbindung, ein Isomer davon, ein pharmazeutisch annehmbares Salz davon, ein Hydrat davon oder ein Solvat davon die Expression von Matrixmetalloprotease-1 (MMP-1) hemmt und in einer Kosmetik-oder Nahrungsmittelzusammensetzung enthalten ist:

[Chemische Formel 1]

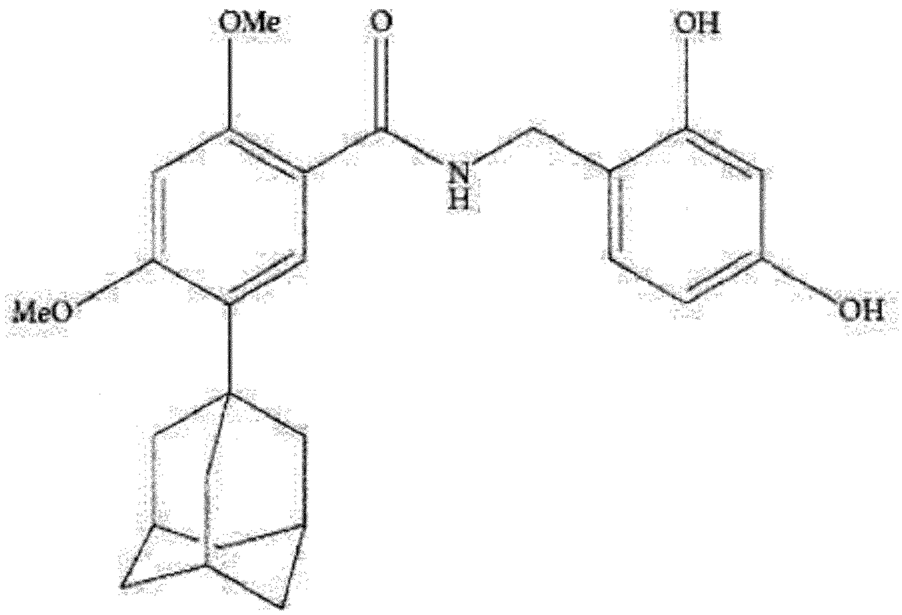

.

2. Nichttherapeutische Verwendung einer Verbindung 5-Adamantan-1-yl-N-(2,4-dihydroxybenzyl)-2,4-dimethoxybenzamid, durch die folgende chemische Formel 1 dargestellt, eines Isomers davon, eines pharmazeutisch annehmbaren Salzes davon, eines Hydrats davon oder eines Solvats davon zur Förderung von Hautelastizität oder Verbesserung von Falten,
wobei die Verbindung, ein Isomer davon, ein pharmazeutisch annehmbares Salz davon, ein Hydrat davon oder ein Solvat davon die Expression von Matrixmetalloprotease-1 (MMP-1) hemmt und in einer Kosmetik-oder Nahrungsmittelzusammensetzung enthalten ist:

[Chemische Formel 1]

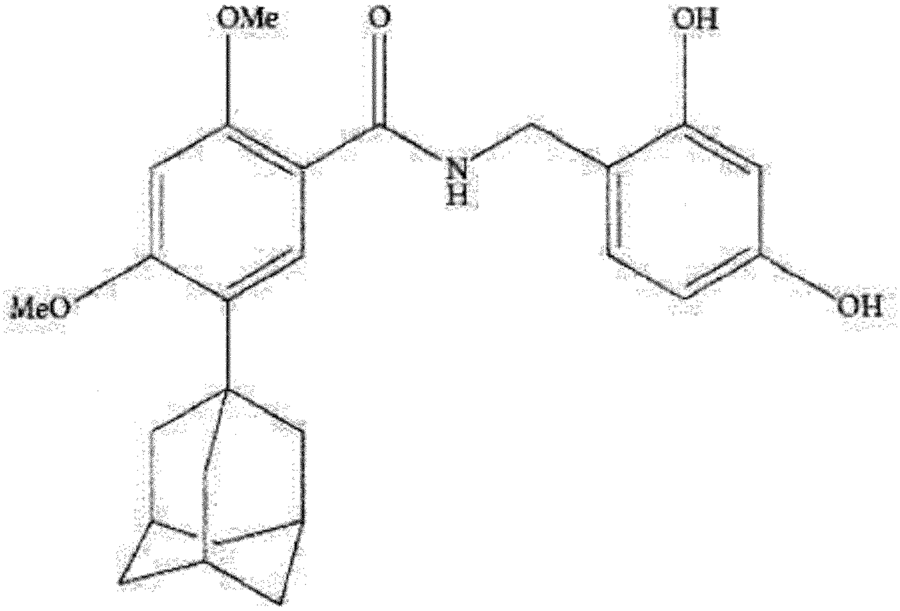

3. Nichttherapeutische Verwendung nach Anspruch 2, wobei die Verbindung, ein Isomer davon, ein pharmazeutisch annehmbares Salz davon, ein Hydrat davon oder ein Solvat davon Kollagen oder Elastin fördert.

4. Nichttherapeutische Verwendung nach Anspruch 2 oder Anspruch 3, wobei die Verbindung, ein Isomer davon, ein pharmazeutisch annehmbares Salz davon, ein Hydrat davon oder ein Solvat davon in einer Konzentration von 0,01 Gew.-% bis 20 Gew.-%, bezogen auf ein Gesamtgewicht der Zusammensetzung, enthalten ist.

## Revendications

1. Utilisation non thérapeutique d'un composé 5-adamantan-1-yl-N-(2,4-dihydroxybenzyl)-2,4-diméthoxybenzamide représenté par la Formule Chimique 1 suivante, d'un isomère de celui-ci, d'un sel pharmaceutiquement acceptable de celui-ci, d'un hydrate de celui-ci, ou d'un solvate de celui-ci pour favoriser l'antivieillissement, dans laquelle le composé, un isomère de celui-ci, un sel pharmaceutiquement acceptable de celui-ci, un hydrate de celui-ci ou un solvate de celui-ci inhibe l'expression de la métalloprotéase matricielle 1 (MMP-1), et est contenu dans une composition cosmétique ou alimentaire.

[Formule Chimique 1]

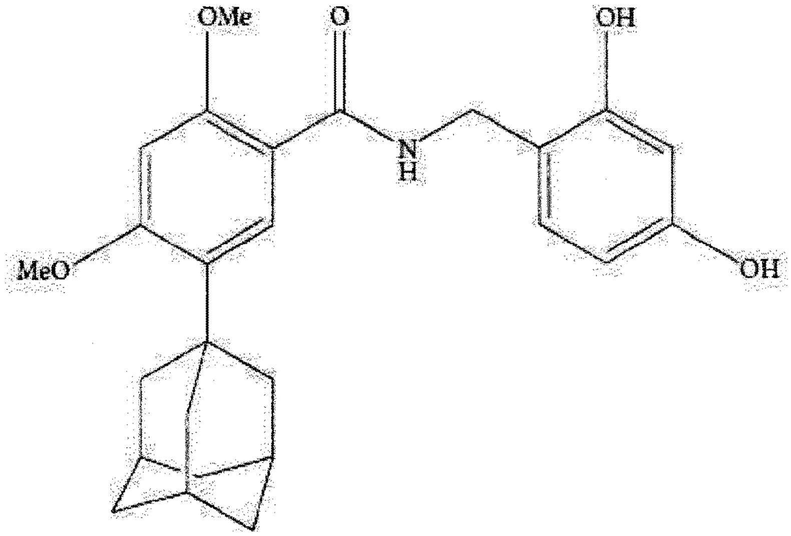

2. Utilisation non thérapeutique d'un composé 5-adamantan-1-yl-N-(2,4-dihydroxybenzyl)-2,4-diméthoxybenzamide représenté par la Formule Chimique 1 suivante, d'un isomère de celui-ci, d'un sel pharmaceutiquement acceptable de celui-ci, d'un hydrate de celui-ci, ou d'un solvate de celui-ci pour améliorer l'élasticité de la peau ou améliorer les rides,
dans laquelle le composé, un isomère de celui-ci, un sel pharmaceutiquement acceptable de celui-ci, un hydrate de celui-ci ou un solvate de celui-ci inhibe l'expression de la métalloprotéase matricielle 1 (MMP-1) et est contenu dans une composition cosmétique ou alimentaire.

[Formule Chimique 1]

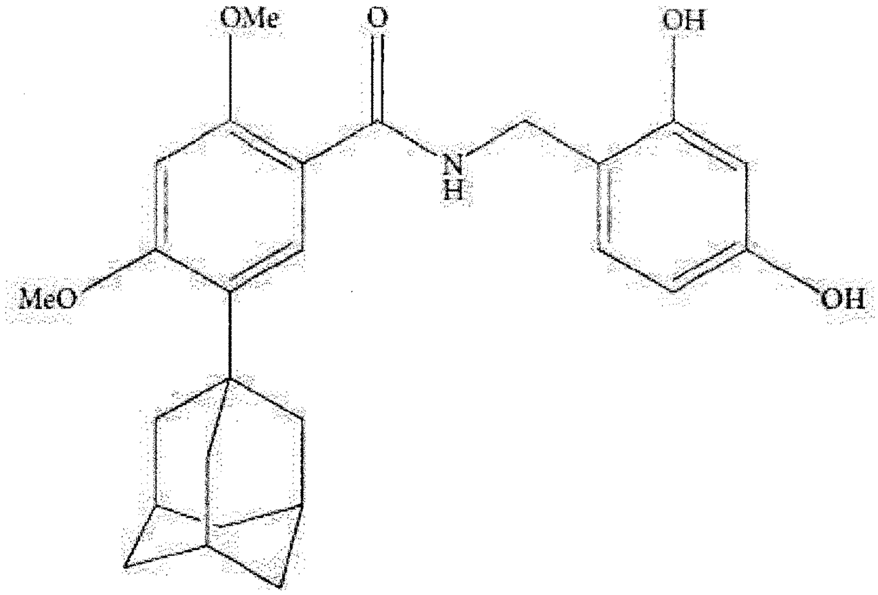

3. Utilisation non thérapeutique selon la revendication 2, dans laquelle le composé, un isomère de celui-ci, un sel pharmaceutiquement acceptable de celui-ci, un hydrate de celui-ci ou un solvate de celui-ci améliore le collagène ou l'élastine.

4. Utilisation non thérapeutique selon la revendication 2 ou 3, dans laquelle le composé, un isomère de celui-ci, un sel pharmaceutiquement acceptable de celui-ci, un hydrate de celui-ci ou un solvate de celui-ci est contenu à une concentration allant de 0,01% en poids à 20% en poids par rapport à un poids total de la composition.

FIG. 1

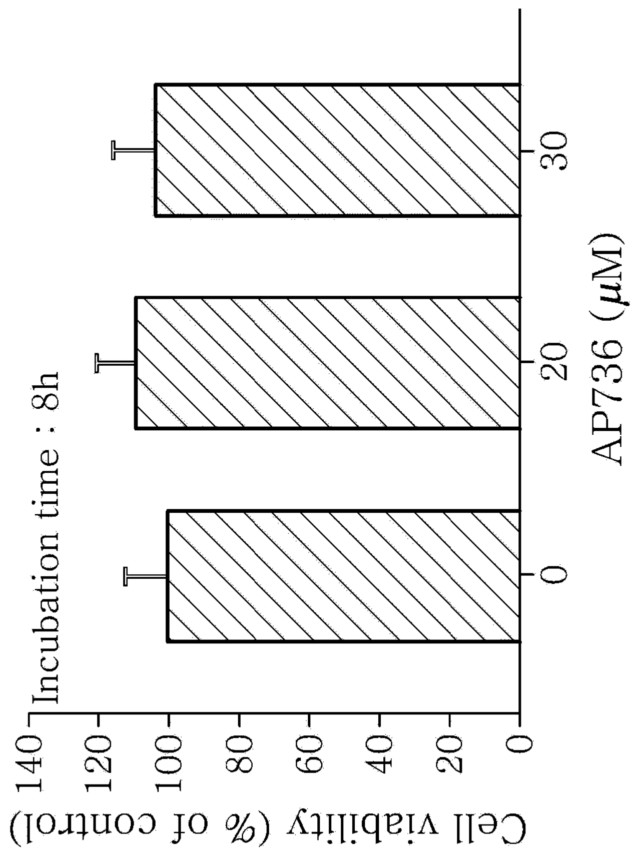
Incubation time : 8h
Cell viability (% of control)
0
20
40
60
80
100
120
140
0
20
30
AP736 (μM)

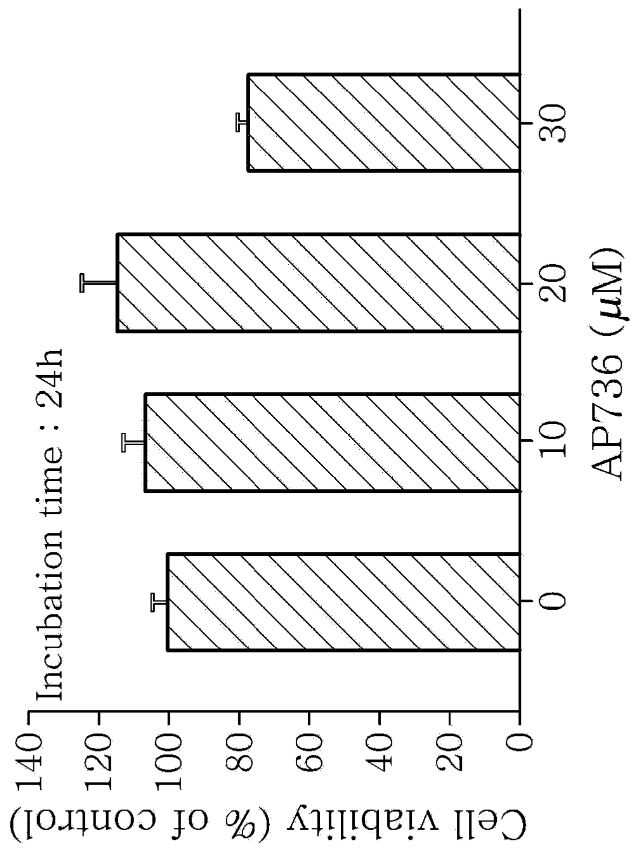
Incubation time : 24h
Cell viability (% of control)
0
20
40
60
80
100
120
140
0
10
20
30
AP736 (μM)

FIG. 2

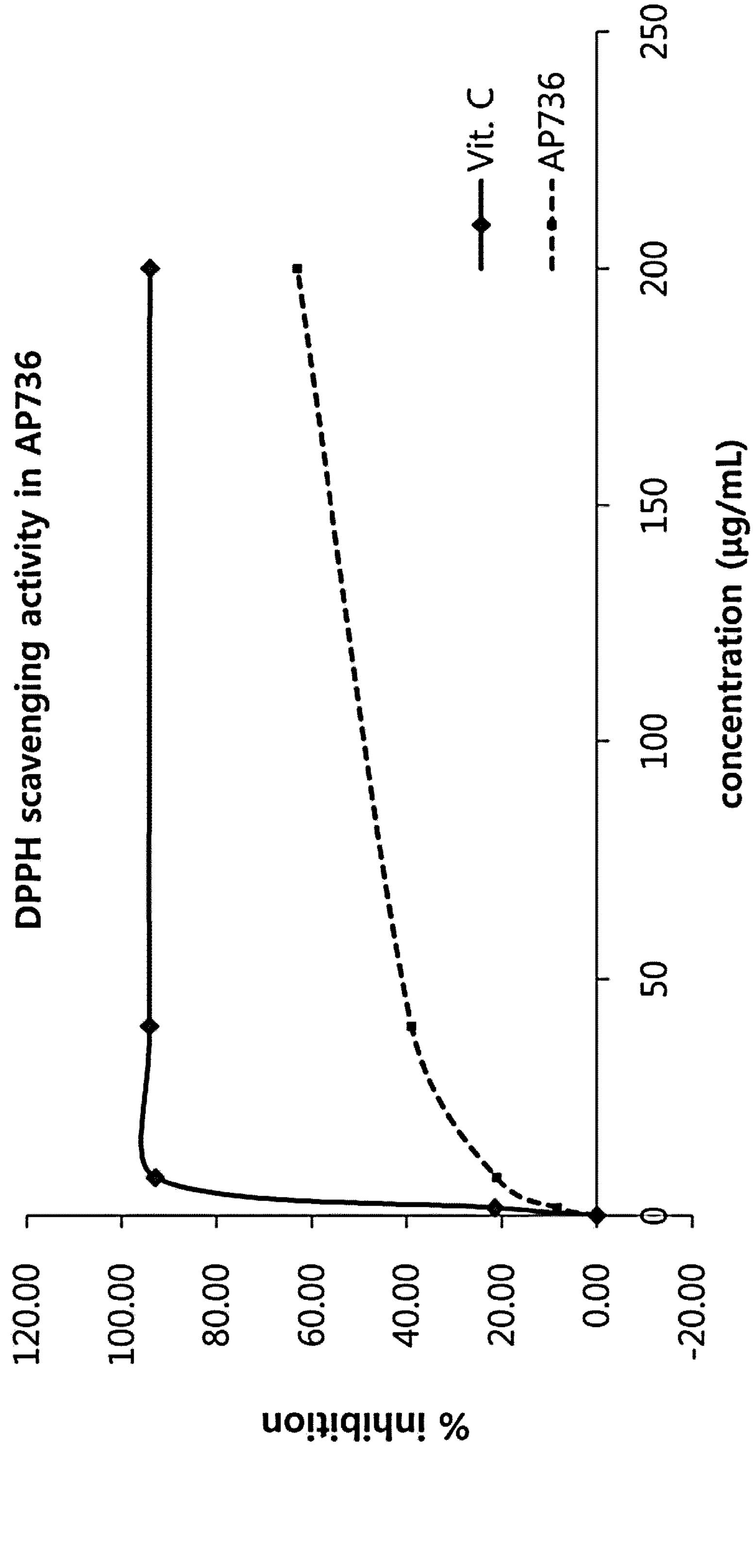
DPPH scavenging activity in AP736
% inhibition
120.00
100.00
80.00
60.00
40.00
20.00
0.00
-20.00
0
50
100
150
200
250
concentration (μg/mL)
Vit. C
AP736

FIG. 3

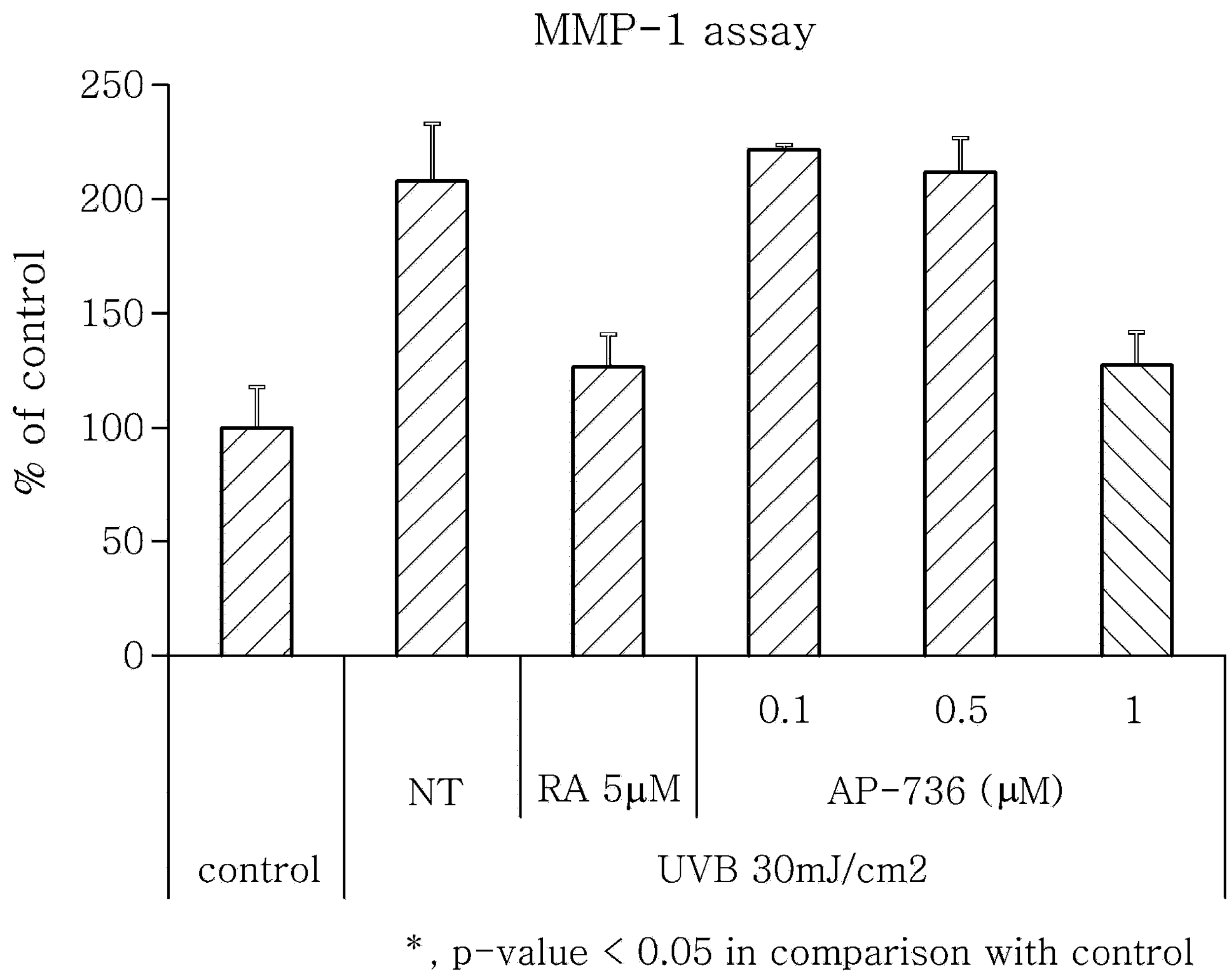
MMP-1 assay
% of control
250
200
150
100
50
0
0.1
0.5
1
NT
RA 5μM
AP-736 (μM)
control
UVB 30mJ/cm2
*, p-value < 0.05 in comparison with control

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014234241 A1
- KR 1020130015954


### Non-patent literature cited in the description


- *Dermatology therapy,* 1998, vol. 16, 357-364
- **LEE et al.** *Experimental Dermatology,* 2013, vol. 22 (11), 762-764
- **HA et al.** *Mediators of Inflammation,* 2014, vol. 2014, 1-11